# FASCICULE DE BREVET EUROPEEN

(11) **EP 0 819 445 B1**
(45) Date de publication et mention de la délivrance du brevet: **07.05.2003**
(21) Numéro de dépôt: 97401733.7
(22) Date de dépôt: 18.07.1997
(51) Int. Cl.: A61N 1/05

(54) **Sonde pour dispositif médical implanté, notamment pour stimulateur cardiaque**
Zuleitung für eine implantierte medizinische Vorrichtung, insbesondere für einen Herzschrittmacher
Lead for an implanted medical device, in particular for a heart pacemaker

(30) Priorité: 19.07.1996 FR 9609068
(43) Date de publication de la demande: 21.01.1998
(73) Titulaire: ELA MEDICAL (Société anonyme), F-92541 Montrouge (FR)
(72) Inventeur: Ollivier, Jean-François, 78280 Guyancourt (FR); Bessoule, Frédéric, 91360 Epinay Sur Orge (FR); Demorgny, Bernard, 92120 Montrouge (FR)
(74) Mandataire: Dupuis-Latour, Dominique

(56) Documents cités:
- EP-A- 0 191 238
- WO-A-91/19533
- FR-A- 2 616 072
- US-A- 4 566 467
- US-A- 5 231 996

## Description

L'invention concerne la fabrication des sondes pour dispositifs médicaux implantés, notamment les sondes de stimulation cardiaque.

Typiquement, ces sondes comportent une gaine isolante creuse en matériau souple avec un conducteur électrique interne (deux conducteurs dans le cas d'une sonde bipolaire), terminée à son extrémité distale par une électrode venant en appui sur la paroi du myocarde.

La réalisation de l'extrémité distale de la sonde consiste principalement à assembler entre eux les trois éléments constitués par le corps cylindrique (également désigné "côté-coeur"), l'électrode de stimulation et le conducteur interne.

L'électrode de stimulation, qui est généralement en carbone poreux, a la forme générale d'un clou, avec une tête aplatie (surface de stimulation) et une tige axiale destinée à permettre la liaison au conducteur. Ce dernier est un élément métallique spiralé et la liaison ― mécanique et électrique ― entre l'électrode et le conducteur est obtenue en enfonçant à force la tige de l'électrode dans la spirale du conducteur ou, alternativement, selon une technique exposée dans le FR-A-2 616 072 au nom d'ELA Médical.

Une fois l'électrode et le conducteur solidarisés, on réalise la liaison mécanique de l'électrode au corps cylindrique par collage, en déposant une certaine quantité de colle dans l'ouverture du corps cylindrique et en enfonçant dans cette dernière la tige de l'électrode jusqu'à ce que la colle vienne légèrement déborder en périphérie de cette ouverture.

Le document EP-A-0191238 expose une sonde endocardiaque pour stimulateur cardiaque, comprenant à son extrémité distale un corps cylindrique creux présentant côté distal une ouverture recevant une électrode de stimulation montée axialement dans cette ouverture, corps et électrode étant mécaniquement solidarisés par collage de paroi en vis-à-vis du corps et de l'électrode, la paroi interne du corps étant conformée de manière à laisser subsister entre électrode et corps, lorsque l'électrode est montée dans le corps, un intervalle périphérique définissant une chambre, cette chambre est une chambre d'expansion de colle comportant un canal de fuite.

Cette technique de montage par collage nécessite cependant un certain doigté pour bien maîtriser la quantité de colle introduite et éviter notamment les débordements, en particulier sur la surface active de l'électrode en carbone poreux, surface qui doit être définie avec précision car c'est elle qui fixe la polarisation pour la stimulation du myocarde.

Il est certes possible, en cas de débordement, d'essuyer le surplus de colle, mais le débordement peut avoir altéré la surface active en carbone poreux de l'électrode ; si le nettoyage du surplus de colle n'est pas correctement nettoyé, il est nécessaire, une fois la colle durcie, de gratter la surface de l'électrode à cet endroit pour mettre à nu le matériau de l'électrode, ce qui peut, ici encore, altérer les qualités de cette surface fonctionnelle.

Lors de l'injection de la goutte de colle, il y a également lieu d'éviter la formation de bulles dans la colle, qui pourraient conduire à une mauvaise solidarisation et/ou une mauvaise étanchéité de la liaison entre électrode et corps cylindrique.

Ces difficultés sont encore accrues dans le cas particulier des sondes comportant, entre électrode et corps cylindrique, un collier intercalaire formé d'un matériau poreux contenant un principe actif tel qu'un stéroïde destiné à être progressivement diffusé dans la région de contact entre électrode et myocarde. Ici encore, un débordement de colle sur la surface fonctionnelle du collier de diffusion risque de réduire la surface de relargage du principe actif et donc de modifier les propriétés fonctionnelles de ce collier.

L'un des buts de la présente invention est de proposer une structure de sonde permettant de maîtriser parfaitement la distribution de la colle au moment de l'injection de celle-ci et d'éviter ainsi les inconvénients précités, notamment en évitant tout risque de débordement de colle sur des surfaces fonctionnelles (ou de cantonner ce débordement à une région très localisée, en tout état de cause non fonctionnelle).

En conservant l'intégrité des surfaces fonctionnelles de l'électrode en carbone poreux et, éventuellement, du collier de diffusion d'un principe actif, on contrôle parfaitement les différents paramètres de la sonde, avec notamment une excellente reproductibilité des performances de celle-ci. Par ailleurs, la disposition des divers éléments exposés ci-après contribue à une meilleure dissociation de la fonction de stimulation et de celle de relargage de la substance médicamenteuse dans le cas d'une sonde à principe actif.

À cet effet, la sonde est du type connu comportant à son extrémité distale un corps cylindrique creux présentant côté distal une ouverture recevant une électrode de stimulation montée axialement dans cette ouverture, corps et électrode étant mécaniquement solidarisés par collage de parois en vis-à-vis du corps et de l'électrode. Selon l'invention, la paroi interne du corps est conformée de manière à laisser subsister entre électrode et corps, lorsque l'électrode est montée dans le corps, un intervalle périphérique définissant une chambre entre des parois internes en vis-à-vis du corps et de l'électrode, cette chambre étant une chambre d'expansion de colle essentiellement fermée par rapport à l'extérieur à l'exception d'un canal d'injection et d'un canal de fuite, de manière que la chambre puisse être progressivement remplie par pénétration de colle sous pression par le canal d'injection et échappement de la colle en excès par le canal de fuite une fois achevé ce remplissage.

De préférence, les canaux d'injection et de fuite sont orientés radialement et débouchent dans la chambre en deux extrémités distinctes de cette dernière.

Dans une première mise en oeuvre, la chambre d'expansion de colle est une chambre annulaire et les canaux d'injection et de fuite débouchent dans cette chambre en des points diamétralement opposés.

Dans une seconde mise en oeuvre, la chambre d'expansion de colle comporte deux chambres annulaires distinctes reliées entre elles par un canal interne de transition, le canal d'injection débouchant dans l'une des chambres et le canal de fuite débouchant dans l'autre chambre, le canal de transition débouchant dans chacune des chambres en un point diamétralement opposé au débouché des canaux d'injection et de fuite, respectivement.

Cette seconde mise en oeuvre s'applique avantageusement au cas où il est prévu entre électrode et corps un collier intercalaire dont une face interne définit une paroi interne de l'une des chambres annulaires, de manière à solidariser également ce collier à l'électrode et/ou au corps par injection de colle dans la chambre d'expansion de colle.

Par ailleurs, on peut prévoir que la chambre d'expansion de colle présente, côté proximal, un espace subsistant entre un conducteur central et le corps cylindrique creux, cet espace ayant une dimension suffisamment réduite pour essentiellement empêcher la pénétration de la colle dans le volume intérieur du corps en direction proximale. Le corps cylindrique creux peut en particulier présenter, à l'endroit dudit espace, une saillie annulaire interne en réduisant localement le diamètre.

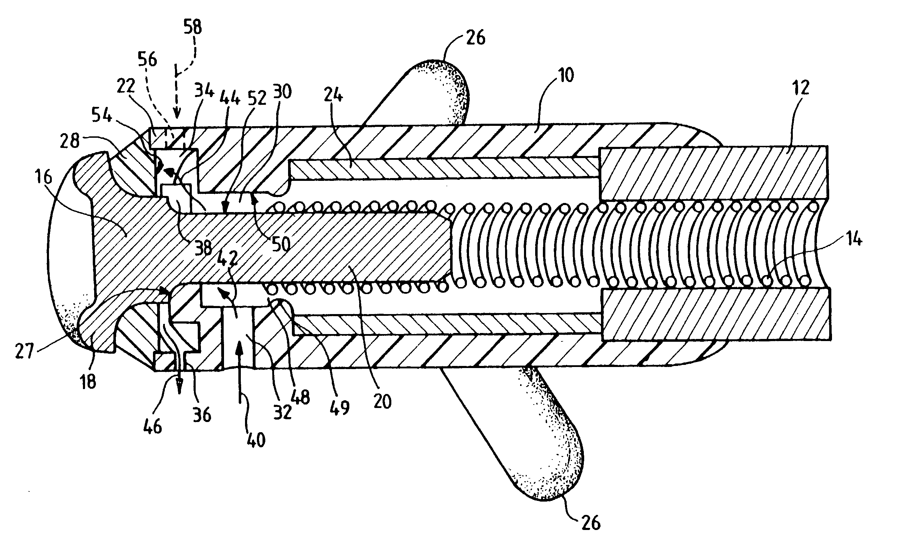

On va maintenant décrire un exemple de réalisation de l'invention, en référence à la figure unique annexée qui est une vue perspective, en coupe, d'une extrémité de sonde selon l'invention.

Sur la figure, la référence 10 désigne le corps cylindrique creux formant l'extrémité distale de la sonde, et monté à l'extrémité d'une gaine isolante flexible 12 enfermant un conducteur métallique spiralé flexible 14 (deux conducteurs dans le cas d'une sonde bipolaire).

Le corps cylindrique 10, tout comme la gaine isolante 12, sont généralement en un matériau biocompatible telle qu'une résine silicone.

La sonde comporte également une électrode de stimulation 16 typiquement en carbone poreux, avec une extrémité frontale aplatie ou tête 18 constituant la surface active proprement dite en contact avec la paroi du myocarde. Cette tête est prolongée par une tige axiale 20 s'étendant à l'intérieur du corps cylindrique creux 10. Le conducteur 14 est relié électriquement et mécaniquement à la tige 20 par enfoncement à force dans la spirale du conducteur 14, qui vient assurer le contact recherché par striction radiale de la tige 20.

Le corps cylindrique 10 comprend à son extrémité distale une ouverture 22 dans laquelle est logée l'électrode 16, centrée par un épaulement 27. Le corps 10 possède également un élément 24 servant de bague radio-opaque en matériau rigide de forme cylindrique, ainsi que des barbes d'ancrage 26 destinées à venir s'insérer dans les trabécules du myocarde au moment de l'implantation de la sonde.

Dans l'exemple illustré, la sonde est également pourvue d'un collier 28 placé autour de l'électrode 16, entre la tête 18 et le débouché 22 du corps cylindrique. Ce collier 28 est généralement réalisé en un silicone poreux chargé d'un principe actif tel qu'un stéroïde destiné à être diffusé dans la région du myocarde au voisinage de l'électrode afin d'améliorer les performances de celle-ci après l'implantation.

Le montage d'une telle sonde consiste, comme on l'a indiqué plus haut, à solidariser entre eux le corps cylindrique 10, l'électrode 16 (qui a été préalablement reliée au conducteur 14) et le collier diffuseur 28, si un tel collier est prévu.

Cette solidarisation est réalisée par collage, et doit être uniforme et étanche, et surtout sans débordement de colle sur les surfaces fonctionnelles de l'électrode (tête 18) et du collier de diffusion 28 (surface externe, libre, de ce collier).

Selon l'invention, il est prévu à cet effet de conformer intérieurement la cavité du corps cylindrique 10 de manière à laisser subsister, entre la tige 20 de l'électrode 16 et le corps cylindrique 10, au-delà (en direction distale) du conducteur 14, une première chambre annulaire 30 entourant complètement la tige 20 de l'électrode 16 ; cette chambre 30 communique avec l'extérieur par un canal radial 32 que l'on désignera par la suite sous le terme de "canal d'injection". On prévoit également une seconde chambre annulaire 34, plus proche de la tête de l'électrode et communiquant avec l'extérieur par un canal 36 que l'on appellera par la suite "canal de fuite".

Les deux chambres 30 et 34 sont isolées entre elles, à l'exception d'un canal de transition 38, par exemple sous forme d'une échancrure radiale réalisée dans l'épaulement 27 de la partie interne du corps cylindrique 10.

Le canal d'injection 32 est situé diamétralement opposé au canal de transition 38, qui est lui-même est situé diamétralement opposé au canal de fuite 36 dans le cas de la présence d'un collier.

L'injection de colle (une colle silicone biocompatible de type classique) est réalisée par une aiguille introduite dans le canal 32. Au fur et à mesure de l'injection, le flux de colle va pénétrer dans le canal d'injection 32 (flèche 40) puis se scinder en deux branches de part et d'autre de la tige de l'électrode (flèche 42).

Les deux fronts de colle qui vont ainsi progresser dans la chambre 30 de part et d'autre de la tige 20 vont ensuite se rejoindre dans la région du canal de transition puis traverser celui-ci (flèche 44) et, à nouveau, se scinder en deux fronts d'avancée de colle dans la seconde chambre 34 de part et d'autre de la tige 20 le long de l'épaulement 27, mais dans une position située plus proche de l'extrémité distale qu'auparavant.

Les deux fronts de colle vont enfin se rejoindre, après avoir fait le tour de la tige 20, au voisinage du canal de fuite 36 où l'opérateur verra la colle sortir (flèche 46) et pourra immédiatement mettre fin à l'injection.

Il est alors certain que le volume prescrit de colle a bien été injecté dans l'extrémité de la sonde, sans bulles ni irrégularités, donc avec une excellente reproductibilité de collage et sans risque d'altération des performances de la sonde du fait de l'absence de débordement sur des surfaces fonctionnelles de la tête d'électrode 18 ou du collier de diffusion 28.

La géométrie particulière des deux chambres 30 et 34 et des canaux d'injection 40, de transition 38 et de fuite 36 permet une progression continue du flux de colle dans l'ensemble de la cavité ainsi constituée, sans formation de bulles, le canal de fuite 36 jouant le rôle d'évent pendant cette opération. On contrôle ainsi parfaitement le volume de colle injecté, grâce au dimensionnement précis des chambres 30 et 34 dont la géométrie n'est liée qu'à la précision de fabrication du corps cylindrique 10 et de l'électrode de stimulation 16. Ces chambres peuvent être dimensionnées de manière à définir parfaitement l'épaisseur du film de colle qui viendra se placer entre les surfaces en vis-à-vis 50 et 52, respectivement du corps cylindrique 10 et de la tige 20 de l'électrode 16, ainsi que de la surface proximale 54 du collier intercalaire 28.

On notera que, côté proximal, l'espace 48 situé entre les dernières spires du conducteur 14 et le corps cylindrique 10 est suffisamment faible pour créer une perte de charge empêchant toute pénétration importante de colle dans le volume intérieur du corps cylindrique en direction proximale, l'essentiel du flux de colle progressant dans la chambre 30 en direction du canal de transition 38. En d'autres termes, la perte de charge résultant du volume de la chambre 30, du canal de transition 38, du volume de la chambre 34 et du canal de fuite 36 est très inférieure à celle de l'intervalle 48 entre l'extrémité du conducteur 14 et la cavité intérieure du corps cylindrique 10 (cet intervalle 48 étant en outre réduit par la présence d'une saillie annulaire interne 49 réduisant localement le diamètre de l'alésage au voisinage de l'extrémité du conducteur 14).

Dans une variante simplifiée de l'invention, notamment pour les sondes ne comportant pas de collier de diffusion, il est possible de prévoir une structure à chambre unique, le corps cylindrique ne comportant dans ce cas que la seconde chambre 34 et l'orifice d'injection étant alors en 56, diamétralement opposé au canal de fuite 36. L'injection se fait alors (flèche 58) par ce canal 56, la colle venant se diviser en deux fronts qui font le tour de la tête de la tige 20 de l'électrode pour se rejoindre (flèche 46) au voisinage du canal de fuite 36.

## Revendications

1. Une sonde pour dispositif médical implanté, notamment pour stimulateur cardiaque, comportant à son extrémité distale un corps cylindrique creux (10) présentant côté distal une ouverture (22) recevant une électrode de stimulation (16) montée axialement dans cette ouverture, corps et électrode étant mécaniquement solidarisés par collage de parois en vis-à-vis du corps et de l'électrode,
la paroi interne du corps étant conformée de manière à laisser subsister entre électrode et corps, lorsque l'électrode est montée dans le corps, un intervalle périphérique définissant une chambre (30 ; 30, 34) entre des parois internes en vis-à-vis (50, 52) du corps et de l'électrode, cette chambre étant une chambre d'expansion de colle essentiellement fermée par rapport à l'extérieur à l'exception d'un canal d'injection (32) et d'un canal de fuite (36), de manière que la chambre puisse être progressivement remplie par pénétration de colle sous pression par le canal d'injection et échappement de la colle en excès par le canal de fuite une fois achevé ce remplissage.

2. La sonde de la revendication 1, dans laquelle les canaux d'injection et de fuite sont orientés radialement et débouchent dans la chambre en deux extrémités distinctes de cette dernière.

3. La sonde de la revendication 1, dans laquelle la chambre d'expansion de colle est une chambre annulaire (34) et les canaux d'injection et de fuite débouchent dans cette chambre en des points diamétralement opposés.

4. La sonde de la revendication 1, dans laquelle la chambre d'expansion de colle comporte deux chambres annulaires distinctes (30, 34) reliées entre elles par un canal interne de transition (38), le canal d'injection débouchant dans l'une des chambres et le canal de fuite débouchant dans l'autre chambre, le canal de transition débouchant dans chacune des chambres en un point diamétralement opposé au débouché des canaux d'injection et de fuite, respectivement.

5. La sonde de la revendication 4, dans laquelle il est prévu entre électrode et corps un collier intercalaire (28) dont une face interne (54) définit une paroi interne de l'une des chambres annulaires, de manière à solidariser également ce collier à l'électrode et/ou au corps par injection de colle dans la chambre d'expansion de colle.

6. La sonde de la revendication 1, dans laquelle, la chambre d'expansion de colle présente, côté proximal, un espace (48) subsistant entre un conducteur central (14) et le corps cylindrique creux (10), cet espace ayant une dimension suffisamment réduite pour essentiellement empêcher la pénétration de la colle dans le volume intérieur du corps en direction proximale.

7. La sonde de la revendication 6, dans laquelle le corps cylindrique creux présente, à l'endroit dudit espace (48), une saillie annulaire interne (49) en réduisant localement le diamètre.

## Claims

1. A lead for an implanted medical device, in particular for a cardiac pacemaker, comprising at its distal end a hollow cylindrical body (10) presenting at its distal side an opening (22) receiving a stimulation electrode (16) axially mounted in this opening, the body and electrode being mechanically fixed together by the gluing of facing walls of the body and of the electrode,
the inner wall of the body being conformed in a manner as to leave between the electrode and the body, when the electrode is mounted in the body, a remaining peripheral space defining a chamber (30; 30, 34) between facing inner walls (50, 52) of the body and of the electrode, this chamber being a glue expansion chamber essentially closed to the exterior except for an injection channel (32) and an escape channel (36), in a manner so that said chamber can be gradually filled by penetration of glue under pressure at the injection channel and escaping of the excess glue by the escape channel once the filling is completed.

2. The lead of claim 1, in which the injection and escape channels are oriented radially and opens in the chamber at two distinct ends of the latter.

3. The lead of claim 1, in which the glue expansion chamber is an annular chamber (34) and the injection and escape channels open in this chamber at diametrically opposed locations.

4. The lead of claim 1, in which the glue expansion chamber comprises two distinct annular chambers (30, 34) connected therebetween by an inner transition channel (38), the injection channel opening in one of these chambers and the escape channel opening in the other chamber, the transition channel opening in each of the chambers at a location diametrically opposite to the outlet of the injection and escape channels, respectively.

5. The lead of claim 4, in which it is provided between the electrode and the body an intermediate ring (28) an inner face (54) of which defines an inner wall of one of the annular chambers, in a manner so as to fix also said ring to the electrode and/or to the body by injection of glue in the glue expansion chamber.

6. The lead of claim 1, in which the glue expansion chamber comprises, at its proximal side, a space (48) left between a central conductor (14) and the hollow cylindrical body (10), said space having a sufficiently small size so as to essentially prevent any penetration of glue in the inner volume of the body in the proximal direction.

7. The lead of claim 6, in which the hollow cylindrical body comprises, at the location of said space (48), an inner annular protrusion (49) which locally reduces the diameter thereof.

## Patentansprüche

1. Eine Sonde für eine implantierte medizinische Vorrichtung, insbesondere für einen Herzschrittmacher, aufweisend an ihrem distalen Ende einen zylindrischen hohlen Körper (10), der auf der distalen Seite eine Öffnung (22) aufweist, welche eine Stimulationselektrode (16) aufnimmt, die axial in dieser Öffnung montiert ist, wobei Körper und Elektrode mechanisch miteinander verbunden sind durch Wandkleben gegenüber dem Körper und der Elektrode,
wobei die innere Wand des Körpers in einer Weise gestaltet ist, dass zwischen der Elektrode und dem Körper, wenn die Elektrode in dem Körper montiert ist, ein peripherer Zwischenraum bestehen bleiben gelassen wird, der eine Kammer (30; 30, 34) definiert zwischen den inneren Wänden (50, 52) gegenüber dem Körper und der Elektrode, wobei diese Kammer eine Kammer für eine Ausdehnung von Klebstoff ist, die im Wesentlichen im Verhältnis zur Außenseite geschlossen ist mit Ausnahme eines Injektionskanals (32) und eines Entweichungskanals (36), derart, dass die Kammer progressiv durch Eindringen von Klebstoff unter Druck durch den Injektionskanal und Austreten des überschüssigen Klebstoffs durch den Entweichungskanal, gefüllt werden kann, sobald diese Auffüllung beendet ist.

2. Sonde nach Anspruch 1, bei welcher der Injektionskanal und der Entweichungskanal radial orientiert sind und in der Kammer an zwei verschiedenen Enden dieser letzteren münden.

3. Sonde nach Anspruch 1, bei welcher die Kammer für eine Ausdehnung von Klebstoff eine ringförmige Kammer (34) ist und der Injektionskanal und der Entweichungskanal in dieser Kammer an diametral gegenüberliegenden Punkten münden.

4. Sonde nach Anspruch 1, bei welcher die Kammer für eine Ausdehnung von Klebstoff zwei verschiedene ringförmige Kammern (30, 34) aufweist, die miteinander durch einen internen Übergangskanal (38) verbunden sind,
wobei der Injektionskanal in einer der Kammern mündet und der Entweichungskanal in der anderen Kammer mündet, wobei der Übergangskanal in jeder der Kammern an einem diametral gegenüberliegenden Punkt zur Mündung des Injektionskanals bzw. des Entweichungskanals mündet

5. Sonde nach Anspruch 4, bei welcher zwischen der Elektrode und dem Körper ein zwischengeschalteter Ring (28) vorgesehen ist, dessen innere Fläche (54) eine innere Wand von der einen der ringförmigen Kammern definiert, derart, dass auch dieser Ring mit der Elektrode und/oder dem Körper durch eine Injektion von Klebstoff in die Kammer für eine Ausdehnung von Klebstoff verbunden wird.

6. Sonde nach Anspruch 1, bei welcher die Kammer für eine Ausdehnung von Klebstoff auf der proximalen Seite einen Raum (48) aufweist, der zwischen einem zentralen Leiter (14) und dem zylindrischen hohlen Körper (10) bestehen bleibt, wobei dieser Raum eine Ausdehnung aufweist, die ausreichend reduziert ist, um das Eindringen des Klebstoffs in das innere Volumen des Körpers in proximaler Richtung im Wesentlichen zu verhindern.

7. Sonde nach Anspruch 6, bei welcher der hohle zylindrische Körper am Ort des Raumes (48) einen inneren ringförmigen Vorsprung (49) aufweist, der lokal den Durchmesser dieses reduziert.
